# FASCICULE DE BREVET EUROPEEN

(11) **EP 1 216 305 B1**
(45) Date de publication et mention de la délivrance du brevet: **30.07.2008**
(21) Numéro de dépôt: 00964296.8
(22) Date de dépôt: 15.09.2000
(51) Int. Cl.: C12N 15/74, C12N 15/63, C12N 15/64, C12N 1/20, C12N 1/21, C12R 1/225

(54) **SOUCHE DE LACTOBACILLUS DELBRUECKII ET SON UTILISATION POUR LE CRIBLAGE DE PLASMIDES**
STAMM VON LACTOBACILLUS DELBRUECKII UND IHRE VERWENDUNG ZUM SCREENING VON PLASMIDEN
LACTOBACILLUS DELBRUCKII STRAIN AND ITS USE FOR SCREENING PLASMIDS

(30) Priorité: 17.09.1999 FR 9911683
(43) Date de publication de la demande: 26.06.2002
(73) Titulaire: INSTITUT NATIONAL DE LA RECHERCHE AGRONOMIQUE (INRA), 75007 Paris Cédex 07 (FR); Compagnie Gervais Danone, 75009 Paris (FR); Rhodia Food S.A., 92408 Courbevoie (FR)
(72) Inventeur: SERROR, Pascale, F-78350 Jouy-en-Josas (FR); FREMAUX, Christophe, F-86000 Poitiers (FR); BENBADIS, Laurent, F-Antony 92160 (FR); MAGUIN, Emmanuelle, F-92150 Montrouge (FR)
(74) Mandataire: Orès, Irène
(86) Numéro de dépôt international: PCT/FR2000/002564
(87) Numéro de publication internationale: WO 2001/021818

(56) Documents cités:
- EP-A- 0 251 064
- EP-A- 0 529 088
- EP-A- 0 603 416
- WO-A-93/18164
- SATOH EIICHI ET AL: "Application of the extracellular alpha-amylase gene from Streptococcus bovis 148 to construction of a secretion vector for yogurt starter strains." APPLIED AND ENVIRONMENTAL MICROBIOLOGY NOV., 1997, vol. 63, no. 11, novembre 1997 (1997-11), pages 4593-4596, XP000921025 ISSN: 0099-2240 cité dans la demande
- DATABASE WPI Section Ch, Week 199333 Derwent Publications Ltd., London, GB; Class D13, AN 1993-260860 XP002141434 -& JP 05 176777 A (MEIJI MILK PROD CO LTD) , 20 juillet 1993 (1993-07-20) cité dans la demande
- ZINK A ET AL: "TRANSFORMATION OF LACTOBACILLUS-DELBRUECKII-SSP-LACTIS BY ELECTROPORATION AND CLONING OF ORIGINS OF REPLICATION BY USE OF A POSITIVE SELECTION VECTOR" FEMS (FEDERATION OF EUROPEAN MICROBIOLOGICAL SOCIETIES) MICROBIOLOGY LETTERS 1991, vol. 78, no. 2-3, 1991, pages 207-212, XP000920937 ISSN: 0378-1097
- KLEIN JUERGEN R ET AL: "Molecular tools for the genetic modification of dairy lactobacilli." SYSTEMATIC AND APPLIED MICROBIOLOGY 1995 (1996), vol. 18, no. 4, 1995, pages 493-503, XP000920944 ISSN: 0723-2020 cité dans la demande
- KLEIN J R ET AL: "CHARACTERIZATION AND SEQUENCE ANALYSIS OF A SMALL CRYPTIC PLASMID FROM LACTOBACILLUS CURVATUS LTH683 AND ITS USE FOR CONSTRUCTION OF NEW LACTOBACILLUS CLONING VECTORS" PLASMID,US,NEW YORK,NY, vol. 30, 1 janvier 1993 (1993-01-01), pages 14-29, XP000606841 ISSN: 0147-619X cité dans la demande
- LEENHOUTS K J ET AL: "LACTOCOCCAL PLASMID PWV01 AS AN INTEGRATION VECTOR FOR LACTOCOCCI" APPLIED AND ENVIRONMENTAL MICROBIOLOGY,US,WASHINGTON,DC, vol. 57, no. 9, 1 janvier 1991 (1991-01-01), pages 2562-2567, XP000564379 ISSN: 0099-2240
- TANNOCK GERALD W ET AL: "Molecular characterization of a plasmid-borne (pGT633) erythromycin resistance determinant (ermGT) from Lactobacillus reuteri 100-63." PLASMID, vol. 31, no. 1, 1994, pages 60-71, XP000921457 ISSN: 0147-619X
- LANGELLA P ET AL: "CONJUGAL TRANSFER OF PLASMID PIP501 FROM LACTOCOCCUS-LACTIS TO LACTOBACILLUS-DELBREUCKII-SSP-BULGARICUS AND LACTOBACILLUS-HELVETICUS" FEMS (FEDERATION OF EUROPEAN MICROBIOLOGICAL SOCIETIES) MICROBIOLOGY, vol. 60, no. 2, 1989, pages 149-152, XP000921466 1989 ISSN: 0378-1097 cité dans la demande
- FONS MICHEL ET AL: "Isolation and characterization of a plasmid from Lactobacillus fermentum conferring erythromycin resistance." PLASMID, vol. 37, no. 3, 1997, pages 199-203, XP000922717 ISSN: 0147-619X

## Description

La présente invention est relative à l'introduction de plasmides dans *Lactobacillus delbrueckii.*

La transformation de bactéries lactiques pour leur permettre d'exprimer des gènes d'intérêt, notamment dans le but d'améliorer les propriétés de ces bactéries dans le cadre de leurs utilisations agroalimentaires, ou de leur conférer la capacité de produire des substances d'intérêt thérapeutique, fait l'objet de nombreuses recherches.

Cependant, les résultats obtenus varient largement selon l'espèce bactérienne concernée. Certaines espèces sont en effet dites « réfractaires » à la transformation, ce qui limite considérablement les possibilités d'introduction et d'expression d'ADN étranger. Parmi les bactéries lactiques réfractaires à la transformation, on mentionnera l'espèce *Lactobacillus delbrueckii*, qui comprend notamment les sous-espèces *Lb*. *delbrueckii* subsp. *bulgaricus, Lb. delbrueckii* subsp. *lactis,* et *Lb. delbrueckii* subsp. *delbrueckii.*

Jusqu'à présent, les essais de transfert de gènes chez *Lb*. *delbrueckii* n'ont donné que peu de résultats ; seuls quelques plasmides capables de s'établir dans cette espèce ont été identifiés. LANGELLA et CHOPIN, [FEMS Microbiol. Lett., 51, 149-52, (1989)] ont décrit l'introduction par conjugaison d'ADN étranger (plasmide conjugatif pIP501 de *Streptococcus agalactiae)* chez *Lb. delbruckii* subsp. *bulgaricus ;* la Demande PCT WO 93/18164 décrit l'introduction chez *Lb. delbruckii* subsp. *bulgaricus* de plasmides qui comprennent le système de réplication du réplicon mutant pVE6002 (CNCM I-1179) également dénommé pG⁺host, dérivé thermosensible (Ts) du plasmide pWVO1 la Demande EP 0603416 au nom de MEIJI MILK PRODUCTS CO LTD décrit l'utilisation du plasmide conjugatif pAMβ1 pour intégrer un gène hétérologue dans le chromosome de *Lb. delbruckii.* BOIZET et al. [Appl. Environ. Microbiol., 54, 3014-3018 (1984)], ont décrit l'introduction d'ADN de bactériophages par transfection de protoplastes de *Lb. delbrueckii* subsp. *bulgaricus.* Dans le cas de la transformation par de l'ADN plasmidique nu, Zink et al. (FEMS Microbiology Letters., 78, 207-212, (1991)) rapportent la première transformation par électroporation d'une souche de *Lb. Delbrückii* subsp. *Lactis* (souche WS97) ; cependant cette souche s'avéra par la suite être une souche de LB. Casei (Klein et al. System. Appl. Microbiol. 18, 493-503, (1995); Tannock et al. (Plasmid., 31, 60-71, (1994)) décrivent l'obtention de transformants *Lb. Delbrückii* 100-14 après électroporation de cette souche à l'aide du plasmide pGT633 issu de *Lb. reuteri ;* la fréquence de transformation observée est de 20 transformants par µg d'ADN plasmidique ; Klein et al. (System. Appl. Microbiol. 18, 493-503, (1995)) rapportent la transformation par électroporation de la souche DSM7290 de *Lb. Delbrückii* subsp. *Lactis* par le plasmide vecteur pCU1882 dérivé d'un plamside de Lb. Curvatus ; la fréquence de transformation rapportée est de 10 par µg d'ADN plasmidique ; la Demande EP 529088, au nom de MEIJI MILK PRODUCTS CO LTD décrit un plasmide dénommé pBUL1, originaire de la souche M-878 de *Lb. delbrueckii* subsp. *bulgaricus* et ses dérivés, utilisables pour transformer *Lb. delbrueckii ;* la Demande JP 5-176777 décrit l'obtention de transformants de *Lb. delbrueckii* par électroporation, en utilisant des plasmides dérivés de pBUL1, ou du plasmide pSYE originaire de *Lactococcus lactis* ; l'électroporation est effectuée après une étape intermédiaire de culture de *Lb*. *delbrueckii* à 42°C pendant 2 à 3 heures dans un milieu LCMG dont le pH initial est compris entre 5 et 5,5 et contenant de l'acide formique (5 à 5,5 mg/ml). Cette étape est suivie de lavages et d'un traitement thermique à des températures variant de 0 à 50°C, pendant 10 minutes à 24 heures. SASAKI et al. [Electrotransformation of Lactobacillus delbrueckii subsp. bulgaricus ; FEMS Microbiol. Rev. 12, 8 (1993)], rapportent la transformation de la souche ATCC11842 et d'une souche dérivée de M-878 de *Lb. delbrueckii* subsp. *bulgaricus* selon la méthode décrite dans la Demande JP 5-176777 ; la fréquence de transformation dans ces conditions est de 1 à 10 transformants par µg d'ADN plasmidique. Elle augmente jusqu'à 6 × 10⁴ transformants par µg d'ADN plasmidique lorsqu'un sous-clone de M-878 (nommé par la suite T-11), déficient dans un système de restriction, est utilisé. La transformation de cette souche (T-11) avec un plasmide dérivé de PSYE1 portant un gène d'α-amylase est également décrite par SATOH et al. [Appl. Environ. Microbiol., 63, 4593-4596, (1997)].

L'identification d'autres plasmides utilisables pour transformer *Lb. delbrueckii* est entravée par le caractère « réfractaire » de cette espèce ; en effet, même dans le cas des plasmides capables de transformer *Lb. delbrueckii* qui sont mentionnés ci-dessus, le taux de transformation demeure généralement faible et la reproductibilité des résultats est irrégulière.

Pour tester la capacité d'autres plasmides à se maintenir sous forme libre ou intégrée chez *Lb*. *delbrueckii*, il est donc souhaitable de disposer d'une souche transformable, et d'une procédure de préparation de cellules compétentes permettant de limiter les échecs dus au caractère réfractaire de *Lb*. *delbrueckii.*

La présente invention s'est donc fixé pour but l'obtention de souches de *Lb*. *delbrueckii* plus facilement transformables, afin de les utiliser comme hôtes permettant la réplication et l'expression d'ADN étranger, ainsi que comme modèles, pour tester des plasmides dans le but de les utiliser ensuite pour transformer d'autres souches de *Lb. delbrueckii,* notamment des souches industrielles.

Une seule souche de *Lb. bulgaricus* est actuellement connue pour permettre des taux de transformation élevés en utilisant de l'ADN plasmidique dont la préparation n'a pas été effectuée à partir de *Lb*. *delbrueckii ;* il s'agit de la souche mutante T-11 mentionnée ci-dessus, qui est déficiente dans un système de restriction/modification. Une telle souche ne constitue toutefois pas un bon modèle pour tester des plasmides destinés à être introduits dans des souches possédant un système de restriction/modification intact.

Les Inventeurs sont parvenus à obtenir, à partir d'une souche représentative de *Lb*. *delbrueckii* subsp. *bulgaricus,* la souche ATCC11842, une souche mutante dont les systèmes de restriction/modification apparaissent similaires à ceux de la souche sauvage ATCC11842 et qui peut être transformée beaucoup plus facilement et de manière plus reproductible que celle-ci. L'utilisation de cette souche mutante leur a permis d'identifier des plasmides potentiellement utilisables pour transformer *Lb. delbrueckii.*

La présente invention a pour objet la souche de *Lb*. *delbrueckii* subsp. *bulgaricus*, dénommée ci-après VI104, déposée selon le Traité de Budapest le 17 septembre 1999, sous le numéro I-2316, auprès de la CNCM (Collection Nationale de Cultures de Microorganismes), 25 rue du Docteur Roux, à Paris.

La présente invention a également pour objet un procédé de criblage de plasmides capables de transformer des bactéries de l'espèce *Lb*. *delbrueckii,* caractérisé en ce que l'on procède à l'électroporation de la souche VI104 en présence d'un plasmide à tester, et à la détection de bactéries transformées.

Dans les bactéries transformées, le plasmide peut être présent sous forme libre, dans le cas d'un plasmide réplicatif, ou sous forme intégrée dans le cas d'un plasmide intégratif.

Avantageusement, des copies dudit plasmide peuvent être récupérées à partir desdites bactéries transformées, afin d'être utilisées pour transformer VI104 ou d'autres souches de *Lb. delbrueckii*, notamment des souches dont les systèmes de restriction sont des barrières à la transformation.

L'électroporation de la souche VI104 peut être effectuée selon les techniques connues de l'art antérieur pour l'électroporation de *Lb*. *delbrueckii,* par exemple celle décrite dans la Demande JP 5-176777. Cependant, les Inventeurs ont observé que l'on obtenait un meilleur rendement de transformation si l'on supprime l'étape intermédiaire de culture mise en oeuvre dans cette méthode, et si l'électroporation est effectuée sur des cultures en début de phase stationnaire, et après un traitement thermique à 45°C entre 10 et 30 min.

La présente invention sera mieux comprise à l'aide du complément de description qui va suivre, qui se réfère à des exemples non-limitatifs d'utilisation de la souche VI104 pour la sélection de plasmides utilisables pour transformer d'autres souches de *Lb*. *delbrueckii.*

### EXEMPLE 1 : COMPARAISON DES PROPRIETES DE RESTRICTION/MODIFICATION DE LA SOUCHE VI104 ET DE LA SOUCHE ATCC11842

Le tableau I ci-dessous montre que les souches ATCC11842 et VI104 restreignent de manière équivalente 3 phages (C5h2, C5, 11) propagés sur les souches CNRZ448 ou CNRZ327.

**TABLEAU I**

| stock phage(souche) | ATCC11842 (pfu/ml) | VI104 (pfu/ml) | Souche propagatrice (pfu/ml) |
|---|---|---|---|
| C5h2 (448) | 7,00E+06 | 1,00E+07 | 2,50E+08 |
| C5h2 (448) | 1.00E+07 | 1,20E+07 | 3,00E+08 |
| C5h2 (448) | 7,70E+05 | 1,40E+06 | 4,00E+07 |
| C5 (448) | 1,50E+03 | 1,10E+05 | 4,20E+07 |
| 11 (327) | 4,50E+06 | 8,00E+06 | 9,00E+07 |

D'autre part, aucune restriction de la part de ATCC11842 ou de VI104 n'est observée pour les phages propagés sur ces souches. Ces résultats indiquent que les systèmes de restriction et de modification mis en jeu dans ces expériences sont comparables dans les deux souches ATCC11842 et VI104.

### EXEMPLE 2 : CONDITIONS DE CULTURE ET DE TRANSFORMATION

Les bactéries VI104 sont cultivées en milieu MRS, 0,1% glycine (DIFCO) jusqu'en début de phase stationnaire. Elles sont ensuite centrifugées et lavées dans du tampon d'électroporation (0,4 M sucrose, 1 mM MgCl₂, 5 mM KH₂PO₄, pH 6,0) et mises en suspension dans ce même tampon à une concentration correspondant à une DO₆₀₀ d'environ 50. La suspension est incubée 20 min à 45°C puis refroidie sur de la glace. Une aliquote de 80 µl de la suspension est mélangée avec l'ADN plasmidique (~1,5 µg) et le mélange est transféré dans une cuve d'électroporation de 0,2 cm. L'électroporation est effectuée à 1 kV, 800 Ω, et 25 µF.

Immédiatement après l'électroporation, le mélange est dilué dans 2 ml de milieu d'expression (0,2 M sucrose, 5% lait écrémé en poudre, 0,1% extrait de levure, 1% casaminoacides, 25 mM MgCl₂). Après incubation pendant 3 h à 37°C dans ce milieu, les cellules sont étalées sur des boites de milieu sélectif (MRS Agar avec 10 µg/ml d'érythromycine ou 10 µg/ml de chloramphénicol selon le marqueur de sélection porté par le plasmide à tester) ; les boites sont incubées en jarre d'anaérobiose à 37°C pendant 48 h, et les colonies résistantes à l'érythromycine ou au chloramphénicol sont sélectionnées pour confirmer la présence d'ADN plasmidique.

### EXEMPLE 3: CRIBLAGE DE PLASMIDES FONCTIONNELS DANS LA SOUCHE VI104

La transformation de VI104 par différents types de plasmides a été effectuée selon le protocole décrit à l'exemple 2 ci-dessus. La présence de formes plasmidiques libres dans les transformants a été établie par extraction d'ADN total [FOUET et SONENSHEIN, J. Bacteriol., 172, 835-44, (1990)], électrophorèse et éventuellement, hybridation de SOUTHERN avec une sonde plasmidique. De l'ADN représentatif de celui utilisé pour la transformation est analysé en parallèle et permet de confirmer l'identité de taille des plasmides.

Les résultats sont illustrés par les Figures 1 à 4 :
Légendes des figures :
   **Figure 1** **:** Résultats de l'analyse électrophorétique d'ADN total de transformants de *L. bulgaricus* VI104. L'ADN total a été préparé à partir des transformants de pJK650 (**A**), de pLEM415 (**B**) et de pGT633 (**C**). Dans chaque cas, les puits 1 et 2 correspondent, respectivement, à l'ADN plasmidique représentatif de celui utilisé lors de la transformation et à l'ADN total d'un transformant.
   **Figure 2** **:** Résultats de l'analyse d'ADN total d'un transformant de *L. bulgaricus* VI104 par pULP8.
      Les puits 1 et 2 correspondent, respectivement, à l'analyse électrophorétique de l'ADN plasmidique représentatif de celui utilisé lors de la transformation et à l'ADN total d'un transformant. Les puits 4 et 5 correspondent, respectivement, à l'hybridation d'une sonde pULP8 contre l'ADN plasmidique représentatif de celui utilisé lors de la transformation et contre l'ADN total d'un transformant après digestions par l'enzyme *Eco*RI.

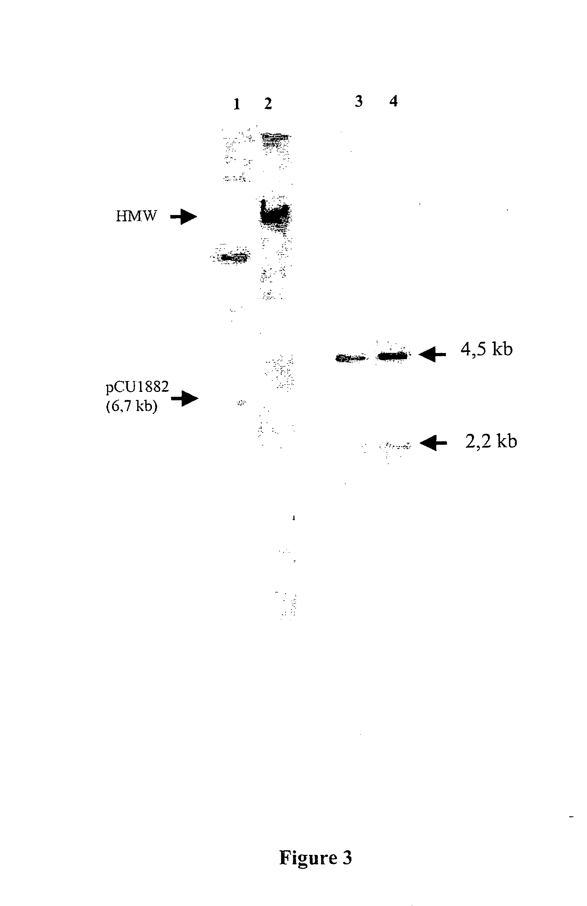

   **Figure 3** : Résultats d'hybridation de l'ADN total des transformants de *L. bulgaricus* VI104 par pCU1882.
      L'ADN total a été préparé à partir des transformants de pCU1882. Les puits 1 et 3 contiennent l'ADN plasmidique représentatif de celui utilisé lors de la transformation et les puits 2 et 4 contiennent l'ADN total d'un transformant. L'ADN est sous forme native dans les puits 1 et 2, et digéré par l'enzyme *Sal*I dans les puits 3 et 4. La sonde utilisée est pCU1882.

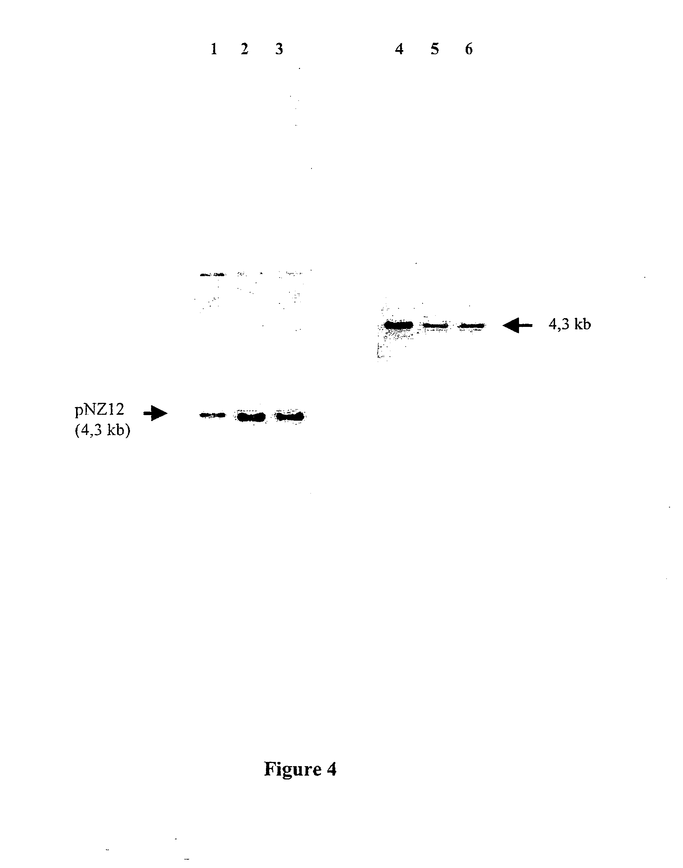

   **Figure 4** : Résultats d'hybridation de l'ADN total de deux transformants de *L. bulgaricus* VI104 par pNZ12.
      Les puits 1 et 4 contiennent l'ADN plasmidique représentatif de celui utilisé lors de la transformation et les puits 2, 3, 5 et 6 contiennent l'ADN total des transformants. L'ADN est sous forme native dans les puits 1 à 3, et digéré par l'enzyme *Nco* I dans les puits 4 à 6. La sonde utilisée est pNZ12.

### 1) Plasmide d'origine homologue.

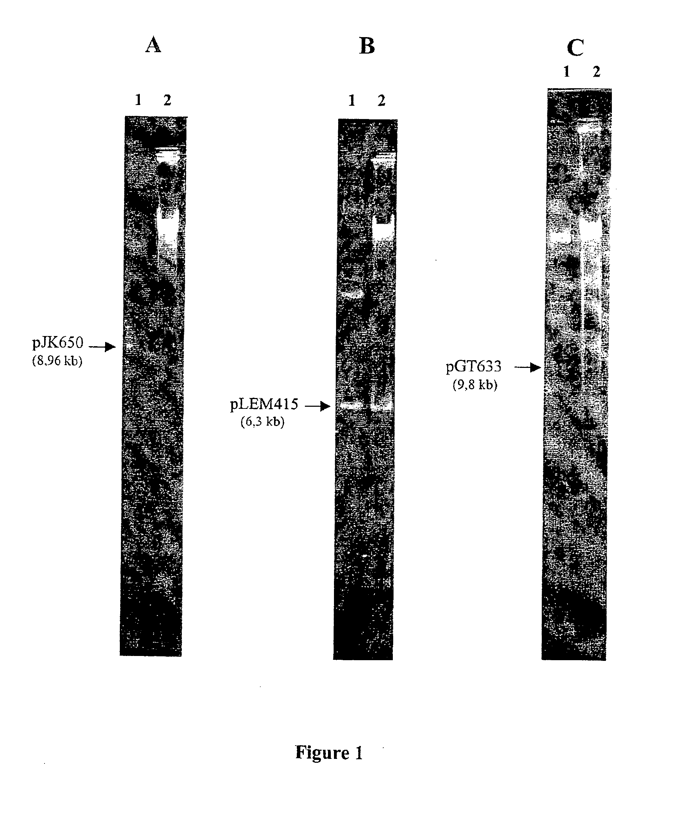

Le plasmide pJK650 (8,96 kb, KLEIN et al., System. Appl. Microbiol. 18, 493-503, 1995) est un dérivé du plasmide pWS58 isolé d'une souche de *Lb. delbrueckii* subsp. *lactis.* Ce plasmide qui porte le gène de résistance à l'érythromycine de pE194 [HORINOUCHI et WEISBLUM, J. Bacteriol., 150, 804-814, (1982)], transforme la souche VI104. La présence de formes libres du plasmide dans les transformants indique que ce plasmide est réplicatif dans VI104 (**Figure 1A**).

### 2) Plasmides d'origine hétérologue.

### Plasmides à réplication de type « cercle roulant ».

Différents plasmides dérivés de réplicons « cercle roulant » ont été testés pour leur capacité à transformer et à se maintenir dans VI104.

Aucun transformant n'a été observé avec les plasmides suivants :
- pE194, plasmide naturellement Ery^{R} originaire de *S. aureus* [HORINOUCHI et WEISBLUM, 1982, publication précitée].

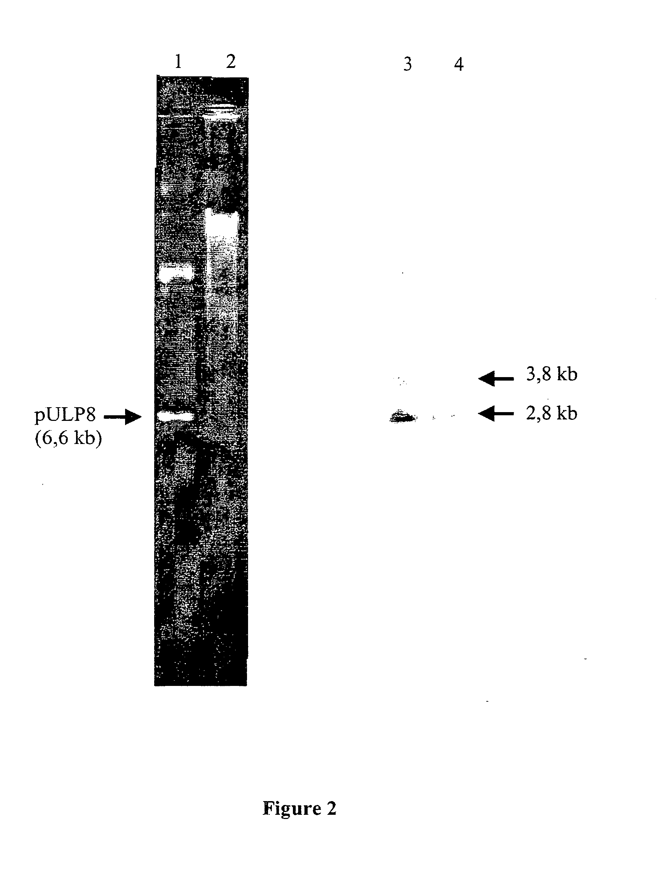

En revanche, des transformants ont été obtenus avec les plasmides suivants :
- pLEM7 [FONS et al., Plasmid, 37, 199-203, (1997)] naturellement Ery^{R} et son dérivé pLEM415 **(****Fig. 1B****).**
- pULP8 (6,6 kb) [BRINGEL et al., Plasmid, 22, 193-202, (1989)], dérivés Ery^{R} (gène de pVA891, [MACRINA et al., Gene, 25, 145-50, (1983)] du plasmide pLPl de *Lactobacillus plantarum* [BOUIA et al., Plasmid, 22, 185-192, (1989)], donnent des transformants avec VI104.L'analyse de transformants de pULP8 **(****Fig. 2****)** a permis de visualiser de formes libres par hybridation avec une sonde radioactive spécifique de pULP8.
- Le plasmide pCU1882 (6,7 kb), dérivé Cm^{R} (gène de pC194) de pLC2 de *L*. *curvatus* LTH683 [KLEIN *et al*., 1993, publication précitée] a conduit à l'obtention de transformants Cm^{R} dans VI104.

L'analyse des transformants a permis de visualiser des formes libres par hybridation avec une sonde radioactive spécifique de pCU1882 (**Fig. 3**).
- Le plasmide pGT633 (9,8 kb), isolé de *Lactobacillus reuteri,* est naturellement Ery^{R} [TANNOCK et al., Plasmid., 31, 60-71, (1994)]. L'analyse des transformants a permis la détection de molécules libres (**Fig. 1C**).
- pNZ12 et pNZ121, dérivés Cm^{R} (gène Cm^{R} de pC194) du plasmide pSH71 de *L*. *lactis* [DE VOS, FEMS Microbiol. Rev., 46, 281-295, (1987)] transforment VI104. L'analyse de transformants de chaque plasmide permet la détection de molécules libres. La Figure 4 illustre les résultats de l'analyse pour pNZ12.

### Plasmides à réplication thêta.

LANGELLA et CHOPIN [1989, publication précitée] ont montré que le plasmide conjugatif pIP501 (30 kb, originaire de *Streptococcus agalactiae*) se réplique à très faible nombre de copies dans *L*. *bulgaricus* (CNRZ431). Du fait de la grande taille de ce plasmide, il n'est pas envisageable de l'utiliser pour électrotransformer VI104. Les Inventeurs ont testé des dérivés de pIP501 non-conjugatifs et de plus petite taille dont pGB305Δ (Ery^{R}, [LE CHATELIER et al., Plasmid, 29, 50-56, (1995)]). Ce plasmide transforme VI104 et se retrouve sous forme libre détectable par analyse de l'ADN total.
- pIL253 (Ery^{R}) est un dérivé non-conjugatif de pAMβ1 [SIMON et CHOPIN, Biochimie, 70, 559-566, (1988)]. Plusieurs transformants ont été obtenus avec VI104. L'extraction d'ADN plasmidique permet de déceler du plasmide libre à faible nombre de copies.

### Vecteur d'intégration site-spécifique.

Le plasmide pMCl contient le site d'attachement *attP* (chevauchant le gène *tRNA^{ser})* et le gène de l'intégrase (*int*) du bactériophage mv4 de *L*. *delbrueckii* [DUPONT et al., J. Bacteriol., 177, 586-595, (1995)]. Ce vecteur s'intègre dans le gène *tRNA^{ser}* d'une grande variété de bactéries lactiques telles que *L*. *plantarum, L*. *casei, L*. *lactis, E. faecalis* et *Streptococcus pneumonia* [AUVRAY et al., J. Bacteriol., 179, 1837-1845, (1997)]. Avec VI104, des intégrants sont obtenus ; l'analyse structurale de leur ADN chromosomique montre que pMC1 s'intègre de manière spécifique.

Ces résultats montrent que divers types de plasmides réplicatifs (plasmides d'origine homologue, plasmides d'origine hétérologue à réplication cercle roulant, plasmides d'origine hétérologue à réplication thêta), ainsi qu'un vecteur intégratif peuvent transformer VI104.

### EXEMPLE 4 : TRANSFORMATION D'AUTRES SOUCHES DE L. DELBRUECKII.

Les plasmides pJK650, pGB305Δ,et pLEM415, qui transforment VI104, ont également été utilisés pour transformer la souche ATCC11842 de *L*. *delbrueckii* subsp. *bulgaricus* et la souche CNRZ327 de *L*. *delbrueckii* subsp. *lactis,* selon le protocole décrit à l'exemple 2 ci-dessus.

Dans ces conditions, des transformants de pJK650 et de pGB305Δ sont obtenus avec les souches ATCC11842 et CNRZ327, des transformants de pLEM415 sont obtenus avec la souche CNRZ327.

## Revendications

1. Souche de *Lb. delbrueckii* subsp. *bulgaricus,* dénommée VI104, déposée le 17 septembre 1999, sous le numéro I-2316, auprès de la CNCM.

2. Procédé de criblage de plasmides capables de transformer des bactéries de l'espèce *Lb*. *delbrueckii,* **caractérisé en ce que** l'on procède à l'électroporation de la souche VI104 selon la revendication 1, en présence du plasmide à tester, et à la détection de bactéries transformées comprenant ledit plasmide.

3. Procédé selon la revendication 2, **caractérisé en ce qu'**il comprend en outre la récupération de copies dudit plasmide à partir desdites bactéries.

## Claims

1. Strain of *Lb. delbrueckii* subsp. *bulgaricus,* called VI104, deposited at the CNCM on 17th September 1999 under number I-2316.

2. Method of screening plasmids capable of transforming bacteria of the species *Lb. delbrueckii,* **characterised in that** strain VI104 according to claim 1 is electroporated in the presence of the plasmid to be tested, and transformed bacteria containing said plasmid are detected.

3. Method according to claim 2, **characterised in that** it further comprises the recovery of copies of said plasmid from said bacteria.

## Patentansprüche

1. Stamm von *Lb. delbrueckii* subsp. *bulgaricus,* bezeichnet mit VI104, der am 17. September 1999 unter der Nummer I-2316 bei der CNCM hinterlegt wurde.

2. Verfahren zur Durchmusterung von Plasmiden, die dazu befähigt sind, Bakterien der Art *Lb. delbrueckii* zu transformieren, **dadurch gekennzeichnet, dass** man den Stamm VI104 nach Anspruch 1 der Elektroporation in Gegenwart des zu untersuchenden Plasmids und der Detektion von Bakterien, die das genannte Plasmid umfassen, unterwirft.

3. Verfahren nach Anspruch 2, **dadurch gekennzeichnet, dass** es außerdem die Gewinnung von Kopien des Plasmids ausgehend von den genannten Bakterien umfasst.
